# EUROPEAN PATENT SPECIFICATION

(11) **EP 0 342 211 B1**
(45) Date of publication and mention of the grant of the patent: **30.03.1994**
(21) Application number: 88909260.7
(22) Date of filing: 28.09.1988
(51) Int. Cl.: A61K 31/405, A23L 1/30

(54) **TREATMENT OF ARTERIOSCLEROSIS BY ADMINISTRATION OF L-TRYPTOPHAN OR L-5-HYDROXYTRYPTOPHAN**
BEHANDLUNG VON ARTERIOSKLEROSIS DURCH VERABREICHUNG VON L-TRYPTOPHANE ODER L-5-HYDROXYTRYPTOPHAN
TRAITEMENT DE L'ARTERIOSCLEROSE PAR ADMINISTRATION DE L-TRYPTOPHANE OU DE L-5-HYDROXYTRYPTOPHANE

(30) Priority: 30.09.1987 US 102723
(43) Date of publication of application: 23.11.1989
(73) Proprietor: FREGLY, Melvin J., Gainesville, FL 32605 (US); CADE, James Robert, Gainesville, FL 32607 (US)
(72) Inventor: FREGLY, Melvin J., Gainesville, FL 32605 (US); CADE, James Robert, Gainesville, FL 32607 (US)
(74) Representative: Hedley, Nicholas James Matthew
(86) International application number: US8803302
(87) International publication number: WO8902737

(56) References cited:
- WO-A-85/03873
- US-A- 4 296 119
- US-A- 4 329 356
- Canadian J. of Physiology and Pharmacology, vol. 65, no. 5, 1987, Melvin J. Fregly et al., pp. 753-764
- J. of Hypertension, vol. 4 (suppl. 6), 1986, F. Squadrito et al., pp. S254-S256
- Chemical Abstracts, vol. 106, no. 19, 11 m y 1987, (Columbus, Ohio, US), M.J. Fregly et al., p. 43, abstract 149203h
- J. of Hypertension, vol. 5, 1987 (London) Melvin J. Fregly et al., pp. 621-628

## Description

### Field of the Invention

The present invention relates to the treatment of atherosclerosis. The work leading to the filing of the present application was supported by Grant HL 29459 from the National Institutes of Health.

### Prior Art

The treatment of high blood pressure has not heretofore been satisfactory. Although some drugs have been found effective to lower blood pressure, the side effects are such that the major question is whether the desirability of lowering blood pressure is outweighed by the detrimental effects of the drug.

The majority of human patients with an elevated blood pressure fall into the categories of borderline or mild hypertension. A significant concern at the present time relates to the necessity of instituting drug therapy in such patients. It is therefore desirable to explore alternative possibilities to control blood pressure, including an understanding of the fundamental processes of nutrition in hypertension. From such understanding, nutritional interventions may then be instituted logically prior to initiation of any drug therapies.

There have been studies which have demonstrated that the acute administration of tryptophan to certain spontaneously hypertensive animals (rats) could lower their blood pressures. [Ito et al, In: Central Nervous System Mechanisms Responsible for Blood Pressure Elevations Induced by p-chlorophenylalanime. J. Pharmac. Exp. Ther. Vol. 181 pp 65-74 (1972); Jarrott et al, In: Serotonin Levels in Vascular Tissue and the Effects of a Serotonin Synthesis Inhibitor on Blood Pressure of Hypertensive Rats, Clin. Exp. Pharmacol. Physiol. Suppl. 2 pp. 201-205 (1975); Fuller et al, In: Antihypertensive Effects of Fluoxitine and L-5-hydroxytryptophan in Rats, Life Sci., Vol. 25, pp. 1237-1242 (1979); Sved et al, In: Studies on the Antihypertensive Action of L-tryptophan, J. Pharmac. Exp. Ther., Vol 221, pp. 329-333 (1982); Wolf et al, In: Effects of L-tryptophan on Blood Pressure in Normotensive and Hypertensive Rats, J. Pharmac. Exp. Ther., Vol 230, pp 324-329 (1984); and U.S. Patent No.4,224,343].

U.S. Patents 3,150,044 and 3,298,916 relate to the treatment of hypertension and report that the administration of L-tryptophan and reserpine act synergistically to produce a lowering of blood pressure.

US-A-4,296,119; US 4,329,35; Can J. Physiol. and Pharmacol., 65(5), (1987) pp 753-764; J. Hypertension, 4 (Suppl 6), 1986), pp. 254-256; Chem. Abs. 106:149203h and J. Hypertension, 5(5), 1987, pp. 621-628 describe the use of L-tryptophan and L-5-hydroxytryptophan in the treatment of hypertension.

The present invention is concerned with the treatment of atherosclerosis to a patient in need thereof.

### SUMMARY OF THE INVENTION

The present invention relates to the use of L-tryptophan and/or L-5-hydroxytryptophan and/or a pharmaceutically acceptable salt thereof as active ingredient(s) in the manufacture of a medicament for the treatment of atherosclerosis in a human or non-human animal.

The medicament may be used for a human patient or non-human animal subjects requiring such treatment The L-tryptophan and/or L-5-hydroxytryptophan is preferably administered in a daily dosage of from about 4 to about 10 grams/day for a human subject (60-150 mg/kg of body weight). It is preferred to administer a dose of 4 to 6 g/day (60-90 mg/kg of body weight) for a human subject.

L-tryptophan may be compounded in capsule, tablet or other orally administrable form utilizing such pharmaceutically acceptable carriers as sugars, e.g., lactose or sucrose, mannitol or sorbitol, cellulose preparations and/or calcium phosphates, as well as binders such as starch, pastes (using maize starch, wheat starch, rice or potato starch); or it may be combined with carriers suitable for parenteral administration [i.v., intra-muscular, etc.] such as push-fit capsules made of gelatin as well as soft, sealed capsules made of gelatin and a plasticizer such as glycerol or sorbitol. The push-fit capsules can contain the active compound in the form of granules, e.g., mixed with filler such as lactose, binders such as starches and/or lubricants such as talc or magnesium stearate and, optimally, stabilizers.

L-tryptophan may be compounded with the above described carriers according to conventional methods.

Alternatively, L-tryptophan or L-5-hydroxytryptophan may be administered as a dietary supplement in certain foods and drinks which are low in protein composition.

## Claims

1. Use of L-tryptophan and/or L-5-hydroxytryptophan and/or a pharmaceutically acceptable salt thereof as active ingredient(s) in the manufacture of a medicament for the treatment of atherosclerosis in a human or non-human animal.

2. Use as claimed in claim 1, wherein the treatment is such that the total daily dosage of the said active ingredient administered is from 60-150 mg/kg body weight/day.

3. Use as claimed in claim 2, wherein the said daily dose is from 60 to 90 mg/kg body weight/day.

4. Use as claimed in any one of claims 1 to 3, wherein the treatment is by oral or parenteral administration of the medicament.

5. Use as claimed in any one of claims 1 to 4, wherein the said medicament is a pharmaceutical composition or a dietary supplement composition.

6. Use as claimed in any one of claims 1 to 5, wherein the treatment is of humans.

## Patentansprüche

1. Verwendung von L-Tryptophan und/oder L-5-Hydroxytryptophan und/oder eines seiner pharmazeutisch verträglichen Salze als wirksamen Bestandteil bei der Herstellung eines Arzneimittels zur Behandlung der Arteriosklerose beim Menschen oder beim Tier.

2. Verwendung nach Anspruch 1, wobei die Behandlung mit einer täglichen Gesamtdosis des verabreichten wirksamen Bestandteils von 60 bis 150 mg/kg Körpergewicht/Tag erfolgt.

3. Verwendung nach Anspruch 2, worin die Tagesdosis 60 bis 90 mg/kg Körpergewicht/Tag beträgt.

4. Verwendung nach einem der Ansprüche 1 bis 3, worin die Behandlung durch orale oder parenterale Verabreichung des Arzneimittels erfolgt.

5. Verwendung nach einem der Ansprüche 1 bis 4, worin das Arzneimittel eine pharmazeutische Zubereitung oder eine ergänzende diätetische Zubereitung ist.

6. Verwendung nach einem der Ansprüche 1 bis 5, wobei die Behandlung am Menschen erfolgt.

## Revendications

1. Utilisation du L-tryptophane et/ou du L-5-hydroxytryptophane et/ou d'un sel pharmaceutiquement acceptable de tels composés comme principe(s) actif(s) dans la fabrication d'un médicament pour le traitement de l'athérosclérose chez l'homme et l'animal autre qu'humain.

2. Utilisation selon la revendication 1, dans laquelle le traitement est tel que le dosage quotidien total en dit principe actif administré est compris entre 60 et 150 mg/kg de poids corporel/jour.

3. Utilisation selon la revendication 2, dans laquelle ladite dose quotidienne est comprise entre 60 et 90 mg/kg de poids corporel/jour.

4. Utilisation selon l'une quelconque des revendications 1 à 3, dans laquelle le traitement est fait par administration orale ou parentérale du médicament.

5. Utilisation selon l'une quelconque des revendication 1 à 4, dans laquelle ledit médicament est une composition pharmaceutique ou une composition de complément diététique.

6. Utilisation selon l'une quelconque des revendications 1 à 5, dans laquelle le traitement est appliqué à l'être humain.
